# EUROPEAN PATENT SPECIFICATION

(11) **EP 2 945 965 B1**
(45) Date of publication and mention of the grant of the patent: **25.07.2018**
(21) Application number: 14703176.9
(22) Date of filing: 13.01.2014
(51) Int. Cl.: C07K 14/47

(54) **PEPTIDE**
PEPTID
PEPTIDE

(30) Priority: 15.01.2013 GB 201300683
(43) Date of publication of application: 25.11.2015
(73) Proprietor: Apitope Technology (Bristol) Limited, Cardiff Bay Cardiff CF10 4PL (GB)
(72) Inventor: WRAITH, David, Bristol BS8 1TD (GB); STREETER, Heather, Bristol BS8 1TD (GB); ORDONEZ, Laurence, 31840 Seilh (FR)
(74) Representative: D Young & Co LLP
(86) International application number: PCT/IB2014/058234
(87) International publication number: WO 2014/111841

(56) References cited:
- WO-A1-96/32957
- WO-A2-03/040165
- WO-A2-2008/055059
- WO-A2-2012/041867
- US-A1- 2007 055 049

### SEQUENCE LISTING

<110> APITOPE INTERNATIONAL NV MERCK SERONO S.A.
<120> PEPTIDE
<130> P044820PCT1
<150> GB1300683.8
   <151> 2013-01-15
<160> 33
<170> PatentIn version 3.5
<210> 1
   <211> 26
   <212> PRT
   <213> Homo sapiens
<400> 1
   His Glu Ala Leu Thr Gly Thr Glu Lys Leu Ile Glu Thr Tyr Phe Ser
   1               5                   10                  15
   Lys Asn Tyr Gln Asp Tyr Glu Tyr Leu Ile
               20                  25
<210> 2
   <211> 28
   <212> PRT
   <213> Homo sapiens
<400> 2
   Thr Trp Thr Thr Cys Gln Ser Ile Ala Phe Pro Ser Lys Thr Ser Ala
   1               5                   10                  15
   Ser Ile Gly Ser Leu Cys Ala Asp Ala Arg Met Tyr
               20                  25
<210> 3
   <211> 28
   <212> PRT
   <213> Homo sapiens
<400> 3
   Gly Val Leu Pro Trp Asn Ala Phe Pro Gly Lys Val Cys Gly Ser Asn
   1               5                   10                  15
   Leu Leu Ser Ile Cys Lys Thr Ala Glu Phe Gln Met
               20                  25
<210> 4
   <211> 19
   <212> PRT
   <213> Homo sapiens
<400> 4
   Leu Thr Gly Thr Glu Lys Leu Ile Glu Thr Tyr Phe Ser Lys Asn Tyr
   1               5                   10                  15
   Gln Asp Tyr
<210> 5
   <211> 19
   <212> PRT
   <213> Homo sapiens
<400> 5
   Trp Thr Thr Cys Gln Ser Ile Ala Phe Pro Ser Lys Thr Ser Ala Ser
   1               5                   10                  15
   Ile Gly Ser
<210> 6
   <211> 15
   <212> PRT
   <213> Homo sapiens
<400> 6
   Val Leu Pro Trp Asn Ala Phe Pro Gly Lys Val Cys Gly Ser Asn
   1               5                   10                  15
<210> 7
   <211> 15
   <212> PRT
   <213> Homo sapiens
<400> 7
   Leu Thr Gly Thr Glu Lys Leu Ile Glu Thr Tyr Phe Ser Lys Asn
   1               5                   10                  15
<210> 8
   <211> 15
   <212> PRT
   <213> Homo sapiens
<400> 8
   Thr Glu Lys Leu Ile Glu Thr Tyr Phe Ser Lys Asn Tyr Gln Asp
   1               5                   10                  15
<210> 9
   <211> 15
   <212> PRT
   <213> Homo sapiens
<400> 9
   Glu Lys Leu Ile Glu Thr Tyr Phe Ser Lys Asn Tyr Gln Asp Tyr
   1               5                   10                  15
<210> 10
   <211> 15
   <212> PRT
   <213> Homo sapiens
<400> 10
   Trp Thr Thr Cys Gln Ser Ile Ala Phe Pro Ser Lys Thr Ser Ala
   1               5                   10                  15
<210> 11
   <211> 15
   <212> PRT
   <213> Homo sapiens
<400> 11
   Thr Thr Cys Gln Ser Ile Ala Phe Pro Ser Lys Thr Ser Ala Ser
   1               5                   10                  15
<210> 12
   <211> 15
   <212> PRT
   <213> Homo sapiens
<400> 12
   Thr Cys Gln Ser Ile Ala Phe Pro Ser Lys Thr Ser Ala Ser Ile
   1               5                   10                  15
<210> 13
   <211> 15
   <212> PRT
   <213> Homo sapiens
<400> 13
   Cys Gln Ser Ile Ala Phe Pro Ser Lys Thr Ser Ala Ser Ile Gly
   1               5                   10                  15
<210> 14
   <211> 15
   <212> PRT
   <213> Homo sapiens
<400> 14
   Gln Ser Ile Ala Phe Pro Ser Lys Thr Ser Ala Ser Ile Gly Ser
   1               5                   10                  15
<210> 15
   <211> 15
   <212> PRT
   <213> Homo sapiens
<400> 15
   Val Leu Pro Trp Asn Ala Phe Pro Gly Lys Val Cys Gly Ser Asn
   1               5                   10                  15
<210> 16
   <211> 276
   <212> PRT
   <213> Homo sapiens
<400> 16
   Gly Leu Leu Glu Cys Cys Ala Arg Cys Leu Val Gly Ala Pro Phe Ala
   1               5                   10                  15
   Ser Leu Val Ala Thr Gly Leu Cys Phe Phe Gly Val Ala Leu Phe Cys
               20                  25                  30
   Gly Cys Gly His Glu Ala Leu Thr Gly Thr Glu Lys Leu Ile Glu Thr
           35                  40                  45
   Tyr Phe Ser Lys Asn Tyr Gln Asp Tyr Glu Tyr Leu Ile Asn Val Ile
       50                  55                  60
   His Ala Phe Gln Tyr Val Ile Tyr Gly Thr Ala Ser Phe Phe Phe Leu
   65                  70                  75                  80
   Tyr Gly Ala Leu Leu Leu Ala Glu Gly Phe Tyr Thr Thr Gly Ala Val
                   85                  90                  95
   Arg Gln Ile Phe Gly Asp Tyr Lys Thr Thr Ile Cys Gly Lys Gly Leu
               100                 105                 110
   Ser Ala Thr Val Thr Gly Gly Gln Lys Gly Arg Gly Ser Arg Gly Gln
           115                 120                 125
   His Gln Ala His Ser Leu Glu Arg Val Cys His Cys Leu Gly Lys Trp
       130                 135                 140
   Leu Gly His Pro Asp Lys Phe Val Gly Ile Thr Tyr Ala Leu Thr Val
   145                 150                 155                 160
   Val Trp Leu Leu Val Phe Ala Cys Ser Ala Val Pro Val Tyr Ile Tyr
                   165                 170                 175
   Phe Asn Thr Trp Thr Thr Cys Gln Ser Ile Ala Phe Pro Ser Lys Thr
               180                 185                 190
   Ser Ala Ser Ile Gly Ser Leu Cys Ala Asp Ala Arg Met Tyr Gly Val
           195                 200                 205
   Leu Pro Trp Asn Ala Phe Pro Gly Lys Val Cys Gly Ser Asn Leu Leu
       210                 215                 220
   Ser Ile Cys Lys Thr Ala Glu Phe Gln Met Thr Phe His Leu Phe Ile
   225                 230                 235                 240
   Ala Ala Phe Val Gly Ala Ala Ala Thr Leu Val Ser Leu Leu Thr Phe
                   245                 250                 255
   Met Ile Ala Ala Thr Tyr Asn Phe Ala Val Leu Lys Leu Met Gly Arg
               260                 265                 270
   Gly Thr Lys Phe
           275
<210> 17
   <211> 26
   <212> PRT
   <213> Homo sapiens
<400> 17
   His Glu Ala Leu Thr Gly Thr Glu Lys Leu Ile Glu Thr Tyr Phe Ser
   1               5                   10                  15
   Lys Asn Tyr Gln Asp Tyr Glu Tyr Leu Ile
               20                  25
<210> 18
   <211> 32
   <212> PRT
   <213> Homo sapiens
<400> 18
   Glu Gly Phe Tyr Thr Thr Gly Ala Val Arg Gln Ile Phe Gly Asp Tyr
   1               5                   10                  15
   Lys Thr Thr Ile Cys Gly Lys Gly Leu Ser Ala Thr Val Thr Gly Gly
               20                  25                  30
<210> 19
   <211> 32
   <212> PRT
   <213> Homo sapiens
<400> 19
   Lys Thr Thr Ile Cys Gly Lys Gly Leu Ser Ala Thr Val Thr Gly Gly
   1               5                   10                  15
   Gln Lys Gly Arg Gly Ser Arg Gly Gln His Gln Ala His Ser Leu Glu
               20                  25                  30
<210> 20
   <211> 32
   <212> PRT
   <213> Homo sapiens
<400> 20
   Gly Gln Lys Gly Arg Gly Ser Arg Gly Gln His Gln Ala His Ser Leu
   1               5                   10                  15
   Glu Arg Val Cys His Cys Leu Gly Lys Trp Leu Gly His Pro Asp Lys
               20                  25                  30
<210> 21
   <211> 28
   <212> PRT
   <213> Homo sapiens
<400> 21
   Thr Trp Thr Thr Cys Gln Ser Ile Ala Phe Pro Ser Lys Thr Ser Ala
   1               5                   10                  15
   Ser Ile Gly Ser Leu Cys Ala Asp Ala Arg Met Tyr
               20                  25
<210> 22
   <211> 28
   <212> PRT
   <213> Homo sapiens
<400> 22
   Thr Ser Ala Ser Ile Gly Ser Leu Cys Ala Asp Ala Arg Met Tyr Gly
   1               5                   10                  15
   Val Leu Pro Trp Asn Ala Phe Pro Gly Lys Val Cys
               20                  25
<210> 23
   <211> 28
   <212> PRT
   <213> Homo sapiens
<400> 23
   Gly Val Leu Pro Trp Asn Ala Phe Pro Gly Lys Val Cys Gly Ser Asn
   1 5 10 15
   Leu Leu Ser Ile Cys Lys Thr Ala Glu Phe Gln Met
   20 25
<210> 24
   <211> 17
   <212> PRT
   <213> Homo sapiens
<400> 24
   Ala Thr Tyr Asn Phe Ala Val Leu Lys Leu Met Gly Arg Gly Thr Lys
   1 5 10 15
   Phe
<210> 25
   <211> 15
   <212> PRT
   <213> Homo sapiens
<400> 25
   His Glu Ala Leu Thr Gly Thr Glu Lys Leu Ile Glu Thr Tyr Phe
   1 5 10 15
<210> 26
   <211> 15
   <212> PRT
   <213> Homo sapiens
<400> 26
   Glu Ala Leu Thr Gly Thr Glu Lys Leu Ile Glu Thr Tyr Phe Ser
   1 5 10 15
<210> 27
   <211> 15
   <212> PRT
   <213> Homo sapiens
<400> 27
   Ala Leu Thr Gly Thr Glu Lys Leu Ile Glu Thr Tyr Phe Ser Lys
   1 5 10 15
<210> 28
   <211> 15
   <212> PRT
   <213> Homo sapiens
<400> 28
   Thr Gly Thr Glu Lys Leu Ile Glu Thr Tyr Phe Ser Lys Asn Tyr
   1 5 10 15
<210> 29
   <211> 15
   <212> PRT
   <213> Homo sapiens
<400> 29
   Gly Thr Glu Lys Leu Ile Glu Thr Tyr Phe Ser Lys Asn Tyr Gln
   1 5 10 15
<210> 30
   <211> 15
   <212> PRT
   <213> Homo sapiens
<400> 30
   Lys Leu Ile Glu Thr Tyr Phe Ser Lys Asn Tyr Gln Asp Tyr Glu
   1 5 10 15
<210> 31
   <211> 15
   <212> PRT
   <213> Homo sapiens
<400> 31
   Leu Ile Glu Thr Tyr Phe Ser Lys Asn Tyr Gln Asp Tyr Glu Tyr
   1 5 10 15
<210> 32
   <211> 15
   <212> PRT
   <213> Homo sapiens
<400> 32
   Ile Glu Thr Tyr Phe Ser Lys Asn Tyr Gln Asp Tyr Glu Tyr Leu
   1 5 10 15
<210> 33
   <211> 15
   <212> PRT
   <213> Homo sapiens
<400> 33
   Glu Thr Tyr Phe Ser Lys Asn Tyr Gln Asp Tyr Glu Tyr Leu Ile
   1 5 10 15

## Description

The present invention relates to peptides from proteolipid protein (PLP). In particular, the invention relates to peptides derivable from one of the hydrophilic regions of PLP which are capable of binding to an MHC molecule and being presented to a T-cell *in vitro* without antigen processing. The invention also relates to the use of such peptides in the treatment and/or prevention of a disease.

### BACKGROUND

Multiple sclerosis (MS) is a chronic degenerative disease affecting the central nervous system, characterized by demyelination of nerve axons. MS may cause numerous physical and mental symptoms, and often progresses to both physical and cognitive disability. Disease onset usually occurs in young adults (20-40 yrs), is more common in women, and affects more than 1 million people around the world.

The disease course of MS is varied and may lie dormant or progress steadily over time. Several subtypes of MS have been described based on patterns of progression. A person with MS can suffer almost any neurological symptom or sign, including changes in sensation such as loss of sensitivity or tingling, pricking or numbness (hypoesthesia and paraesthesia), muscle weakness, clonus, muscle spasms, or difficulty in moving; difficulties with coordination and balance (ataxia); problems in speech (dysarthria) or swallowing (dysphagia), visual problems (nystagmus, optic neuritis including phosphenes, or diplopia), fatigue, acute or chronic pain, and bladder and bowel difficulties.

MS is currently believed to be an immune-mediated disorder in which the body's own immune system attacks and damages myelin.

There is no known cure for MS. Several current therapies have proven beneficial in restoring function after an attack (relapse), preventing or reducing the degree or frequency of new attacks (relapses), or preventing or reducing the extent of disability. However, many current MS therapies have been associated with adverse effects or are poorly tolerated. There is thus a need for alternative therapies for MS which are effective at treating MS and at alleviating or reducing the symptoms of MS.

### SUMMARY OF THE INVENTION

The present inventors have identified a number of peptides derivable from proteolipid protein (PLP) which can be presented by fixed antigen presenting cells to T-cells. These peptides may be useful in the prevention and/or treatment of demyelinating diseases such as multiple sclerosis.

In a first aspect, therefore, the present invention provides a peptide which is capable of binding to an MHC molecule *in vitro* and being presented to a T cell without antigen processing, and which is selected from the following PLP peptides:
PLP 39-53: LTGTEKLIETYFSKN (SEQ ID NO. 7)
PLP 42-56: TEKLIETYFSKNYQD (SEQ ID NO. 8) and
PLP 43-57: EKLIETYFSKNYQDY (SEQ ID NO. 9).

In a second aspect there is provided a peptide according to the first aspect of the invention for use in the treatment and/or prevention of a demyelinating disease.

In a third aspect, the present invention provides a pharmaceutical composition comprising one or more peptide(s) according to the first aspect of the invention.

Described herein is a method for treating and/or preventing a demyelinating disease in a subject in need of same which comprises the step of administering a peptide according to the first aspect of the invention to the subject.

Described herein is the use of a peptide according to the first aspect of the invention in the manufacture of a medicament for use in the prevention and/or treatment of a demyelinating disease.

In connection with the second aspect of the invention, the disease may be multiple sclerosis.

### BRIEF DESCRIPTION OF THE FIGURES

Figure 1 - **Figure 1****:** Three PLP peptide regions respond *in vitro* and *in vivo* and the response correlates to the DR2 binding prediction. A, represents in broad outline the binding prediction of the PLP peptides to HLA-DRB1*1501 in silico (IEDB and NetMHCII methods) and the ability of these peptides to induce a proliferative response in these mice. B and C represents the ability of 4 of these long peptides to induce EAE (2 individual experiments).

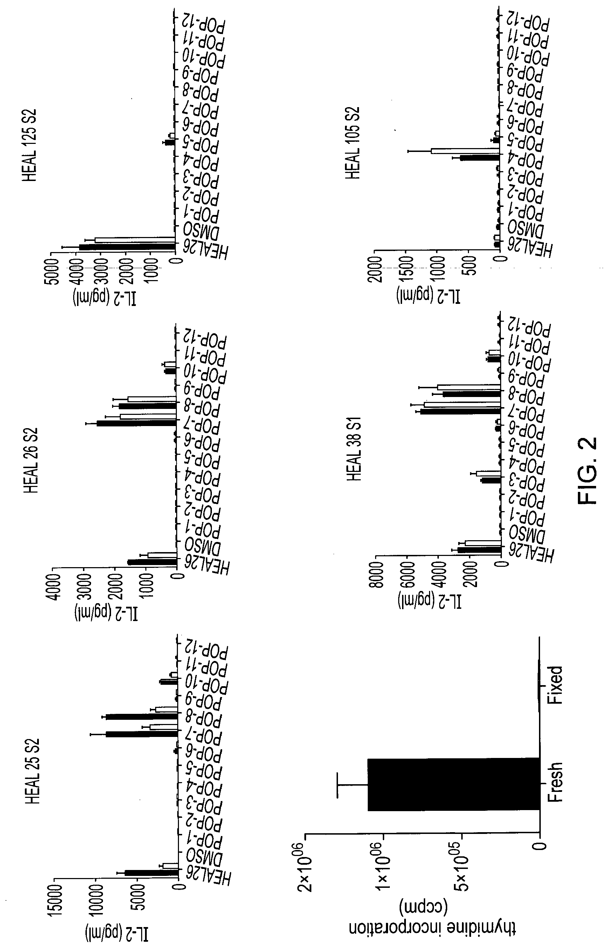

figure 2- - Identification of apitopes within HEAL-26

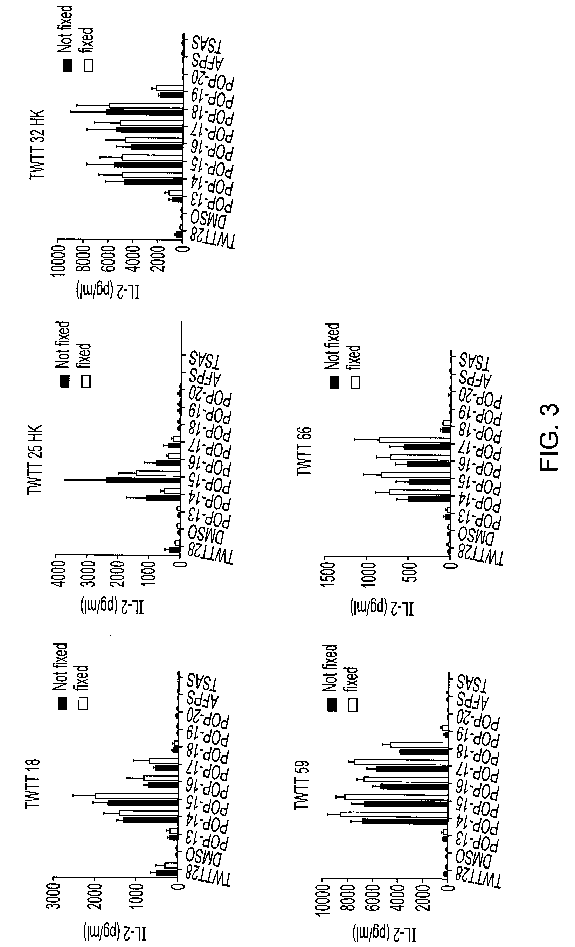

Figure 3 - Identification of apitopes within TWTT-28

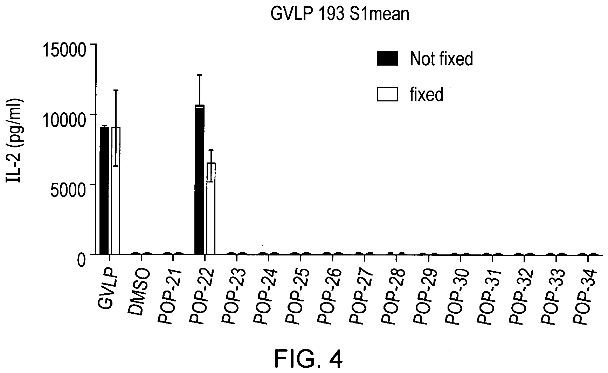

Figure 4 - Identification of apitopes within GVLP-28
Figure 5 - Tolerisation protocol
Figure 6 - Proliferation and cytokine production of LNC from mice pre-treated with HEAL-26 or POP-4. A, represents means +/- standard error of the mean (SEM) of thymidine incorporation of LNC from mice pre-treated with HEAL-26 or POP-4 apitope.
Figure 7 - Proliferation and cytokine production of SPL from mice pre-treated with HEAL-26 or POP-4. A, represents means +/- SEM of thymidine incorporation of SPL from mice pre-treated with HEAL-26 or POP-4 apitope.
Figure 8 - Proliferation and cytokine production of LNC from mice pre-treated with TWTT-28. A, represents means +/- SEM of thymidine incorporation of LNC from mice pre-treated with TWTT-28 apitope.
Figure 9 - Proliferation and cytokine production of SPL from mice pre-treated with TWTT-28. A, represents means +/- SEM of thymidine incorporation of SPL from mice pre-treated with TWTT-28 apitope.
Figure 10 - Proliferation and cytokine production of LNC from mice pre-treated with POP-15. A, represents means +/- SEM of thymidine incorporation of LNC from mice pre-treated with POP-15 apitope.
Figure 11 - Proliferation and cytokine production of SPL from mice pre-treated with POP-15. A, represents means +/- SEM of thymidine incorporation of SPL from mice pre-treated with POP-15 apitope.
Figure 12 - Proliferation and cytokine production of LNC from mice pre-treated with POP-22. A, represents means +/- SEM of thymidine incorporation of LNC from mice pre-treated with POP-22 apitope.
Figure 13 - Proliferation of SPL from mice pre-treated with POP-22. A, represents means +/- SEM of thymidine incorporation of SPL from mice pre-treated with POP-22 apitope.
Figure 14 - Proliferation and cytokine production of LNC from mice pre-treated with POP-22 or GVLP-28. A, represents means +/- SEM of thymidine incorporation of LNC from mice pre-treated with POP-22 (left panel) or GVLP-28 (right panel) apitope.
Figure 15 - Proliferation and cytokine production of SPL from mice pre-treated with POP-22 and GVLP-28. A, represents means +/- SEM of thymidine incorporation of SPL from mice pre-treated with POP-22 or GVLP-28 apitope.

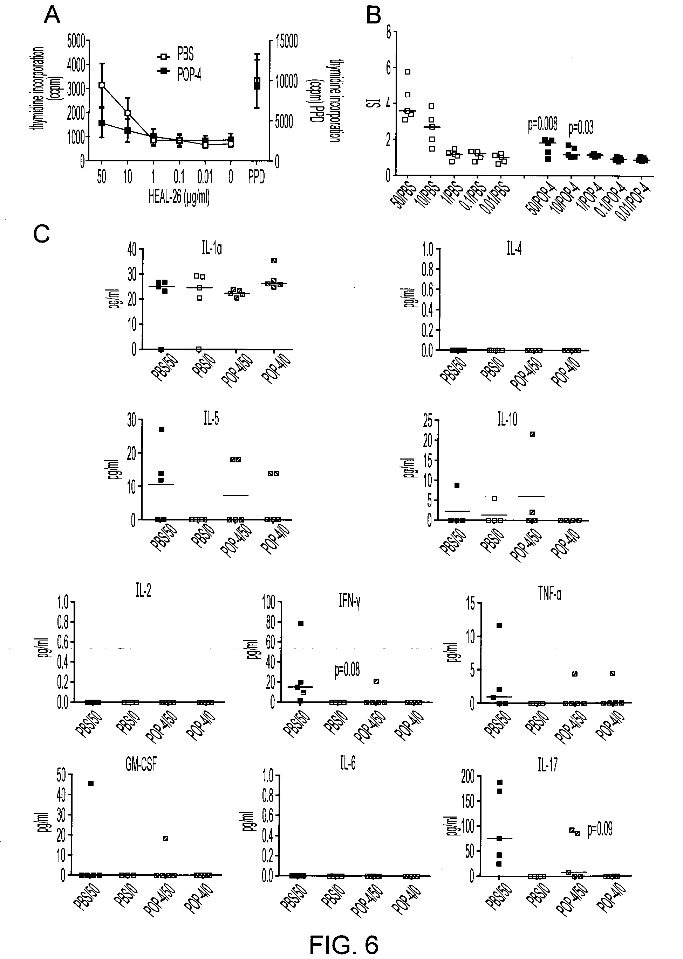

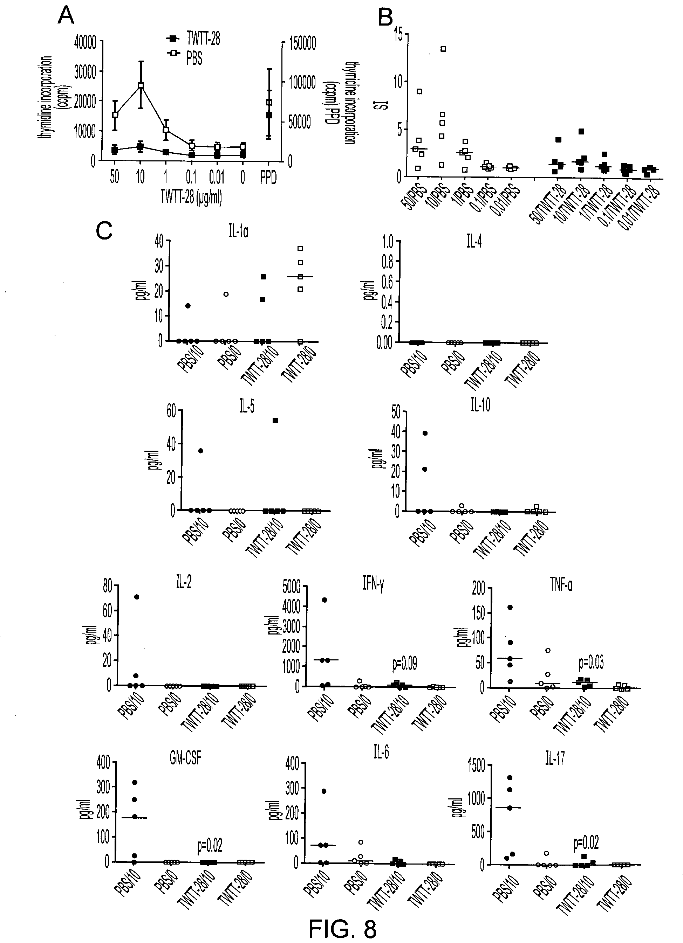

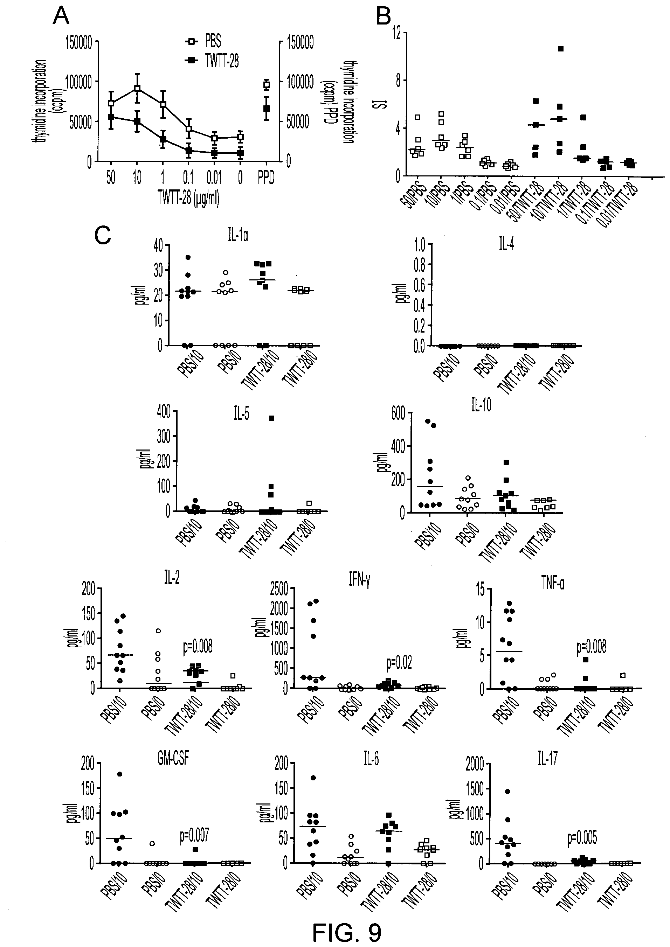

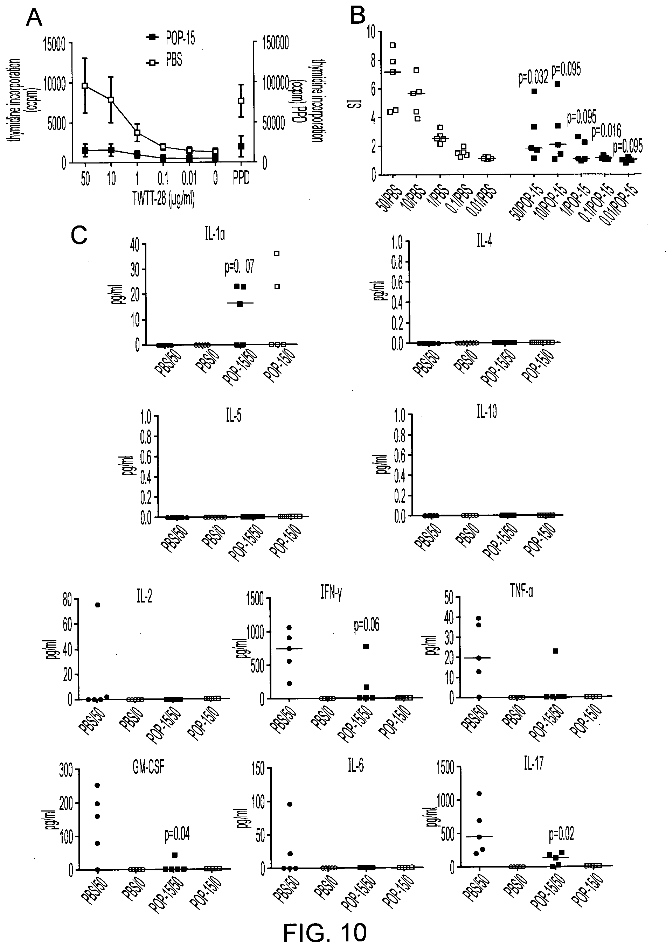

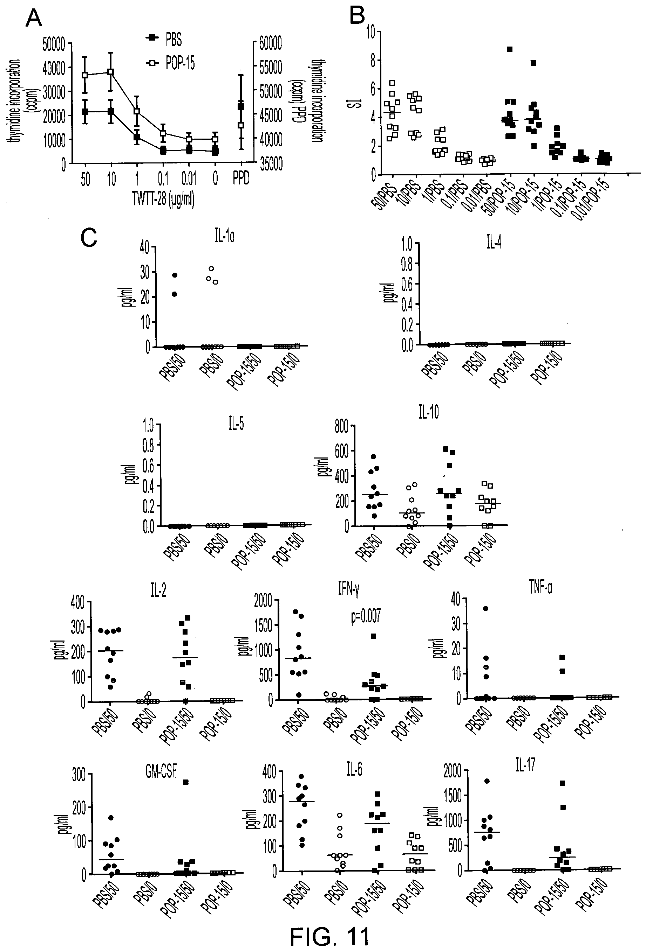

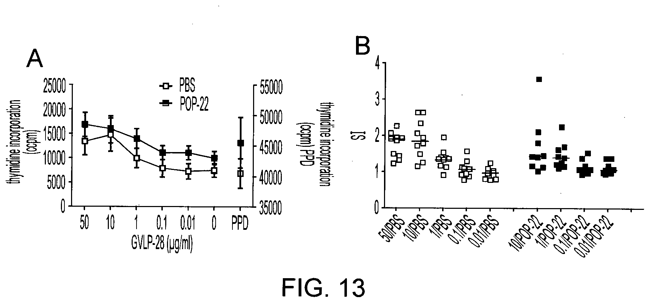

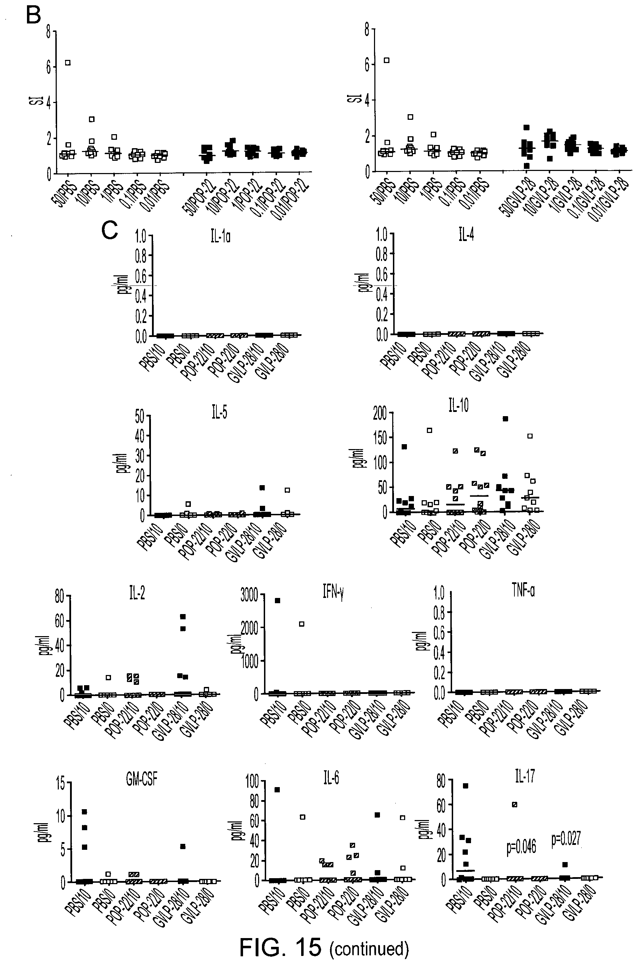

For each of Figures 6 to 15, B, shows the stimulation index of thymidine incorporation. C, represents the amount of cytokine secreted in the culture supernatant. In B and C, each point per dose represents an individual mouse and the bar represents the median. p was calculated using Mann-Whitney test. Only the low p values are shown. A p value <.05 is considered significant.

### DETAILED DESCRIPTION

In a first aspect, the present invention relates to a peptide.

### Peptides

The term "peptide" is used in the normal sense to mean a series of residues, typically L-amino acids, connected one to the other typically by peptide bonds between the α-amino and carboxyl groups of adjacent amino acids The term includes modified peptides and synthetic peptide analogues.

A peptide of the present invention is of a length that is capable of binding to a major histocompatibility complex (MHC) class I or II molecule *in vitro* and being presented to a T cell without antigen processing. In other words the peptides are capable of binding directly into the peptide binding groove of the MHC molecule without requiring any trimming at one or both ends.

Peptides that bind to MHC class I molecules are typically 7 to 13, more usually 8 to 10 amino acids in length. The binding of the peptide is stabilised at its two ends by contacts between atoms in the main chain of the peptide and invariant sites in the peptide-binding groove of all MHC class I molecules. There are invariant sites at both ends of the groove which bind the amino and carboxy termini of the peptide. Variations in peptide length are accommodated by a kinking in the peptide backbone, often at proline or glycine residues that allow the required flexibility.

Peptides which bind to MHC class II molecules are typically between 8 and 20 amino acids in length, more usually between 10 and 17 amino acids in length, and can be much longer. These peptides lie in an extended conformation along the MHC II peptide-binding groove which (unlike the MHC class I peptide-binding groove) is open at both ends. The peptide is held in place mainly by main-chain atom contacts with conserved residues that line the peptide-binding groove.

The peptide of the present invention may be made using chemical methods (Peptide Chemistry, A practical Textbook. Mikos Bodansky, Springer-Verlag, Berlin.). For example, peptides can be synthesized by solid phase techniques (Roberge JY et al (1995) Science 269: 202-204), cleaved from the resin, and purified by preparative high performance liquid chromatography (e.g., Creighton (1983) Proteins Structures And Molecular Principles, WH Freeman and Co, New York NY). Automated synthesis may be achieved, for example, using the ABI 43 1 A Peptide Synthesizer (Perkin Elmer) in accordance with the instructions provided by the manufacturer.

The peptide may alternatively be made by recombinant means or by cleavage from a longer polypeptide. For example, the peptide may be obtained by cleavage from myelin proteolipid protein. The composition of a peptide may be confirmed by amino acid analysis or sequencing (e.g., the Edman degradation procedure).

### Myelin proteolipid protein (PLP)

Myelin is a dielectric (electrically insulating) material that forms a layer, the myelin sheath, usually around only the axon of a neuron. It is essential for the proper functioning of the nervous system. Some of the proteins that make up myelin are myelin basic protein (MBP), myelin oligodendrocyte glycoprotein (MOG), and proteolipid protein (PLP).

Myelin proteolipid protein (PLP), the most abundant protein of central nervous system (CNS) myelin, is a hydrophobic integral membrane protein.

The sequence of human PLP is shown in SEQ ID No. 16
SEQ ID No. 16

The peptide of the invention is derivable from a hydrophilic region of the PLP sequence. The peptide may be derivable from a fragment of the antigen which arises by natural processing of the antigen by an antigen presenting cell.

The hydrophilic regions of PLP are:
PLP 36-61: **HEAL**TGTEKLIETYFSKNYQDYEYLI (SEQ ID No. 17)
PLP 88-119: **EGFY**TTGAVRQIFGDYKTTICGKGLSATVTGG (SEQ ID No. 18)
PLP 104-135: **KTTI**CGKGLSATVTGGQKGRGSRGQHQAHSLE (SEQ ID No. 19)
PLP 119-150: **GQKG**RGSRGQHQAHSLERVCHCLGKWLGHPDK (SEQ ID No. 20)
PLP 179-206: **TWTT**CQSIAFPSKTSASIGSLCADARMY (SEQ ID No. 21)
PLP 192-219: **TSAS**IGSLCADARMYGVLPWNAFPGKVC (SEQ ID No. 22)
PLP 207-234: **GVLP**WNAFPGKVCGSNLLSICKTAEFQM (SEQ ID No. 23)
PLP 260-276: **ATYN**FAVLKLMGRGTKF (SEQ ID No. 24)

The peptide comprises a portion of the following proteolipid protein (PLP) region:
PLP 36-61: HEALTGTEKLIETYFSKNYQDYEYLI (SEQ ID NO. 1)

The peptide comprises a portion of the following region:
PLP 39-57: LTGTEKLIETYFSKNYQDY (SEQ ID NO. 4)

The peptide is selected from the following PLP peptides:
PLP 39-53: LTGTEKLIETYFSKN (SEQ ID NO. 7)
PLP 42-56: TEKLIETYFSKNYQD (SEQ ID NO. 8) and
PLP 43-57: EKLIETYFSKNYQDY (SEQ ID NO. 9).

### Apitopes

In an adaptive immune response, T lymphocytes are capable of recognising internal epitopes of a protein antigen. Antigen presenting cells (APC) take up protein antigens and degrade them into short peptide fragments. A peptide may bind to a major histocompatibility complex (MHC) class I or II molecule inside the cell and be carried to the cell surface. When presented at the cell surface in conjunction with an MHC molecule, the peptide may be recognised by a T cell (via the T cell receptor (TCR)), in which case the peptide is a T cell epitope.

T cell epitopes play a central role in the adaptive immune response to any antigen, whether self or foreign. The central role played by T cell epitopes in hypersensitivity diseases (which include allergy, autoimmune diseases and transplant rejection) has been demonstrated through the use of experimental models. It is possible to induce inflammatory or allergic diseases by injection of synthetic peptides (based on the structure of T cell epitopes) in combination with adjuvant.

By contrast, it has been shown to be possible to induce immunological tolerance towards particular peptide epitopes by administration of peptide epitopes in soluble form.

Administration of soluble peptide antigens has been demonstrated as an effective means of inhibiting disease in experimental autoimmune encephalomyelitis (EAE - a model for multiple sclerosis (MS)) (Metzler and Wraith (1993) Int. Immunol. 5:1159-1165; Liu and Wraith (1995) Int. Immunol. 7:1255-1263; Anderton and Wraith (1998) Eur. J. Immunol. 28:1251-1261); and experimental models of arthritis, diabetes, and uveoretinitis (reviewed in Anderton and Wraith (1998) as above). This has also been demonstrated as a means of treating an ongoing disease in EAE (Anderton and Wraith (1998) as above).

The use of tolerogenic peptides to treat or prevent disease has attracted considerable attention. One reason for this is that it has been shown that certain tolerogenic epitopes can down-regulate responses of T cells for distinct antigens within the same tissue. This phenomenon, known as "bystander suppression" means that it should be possible to induce tolerance to more than one epitope (preferably all epitopes) within a given antigen, and to more than one antigen for a given disease, using a particular tolerogenic peptide (Anderton and Wraith (1998) as above). This would obviate the need to identify all of the pathogenic antigens within a particular disease.

Peptides are also a favourable option for therapy because of their relatively low cost and the fact that peptide analogues can be produced with altered immunological properties. Peptides may thus be modified to alter their interactions with either MHC or TCR.

One possible problem with this approach is that it has been shown that not all peptides which act as T cell epitopes are capable of inducing tolerance. The myelin basic protein (MBP) peptide 89-101 is an immunodominant antigen after immunisation and is also a very effective immunogenic both in terms of priming for T cell reactivity and induction of EAE. However, this peptide has been shown to be ineffective at inducing tolerance when administered in solution (Anderton and Wraith (1998), as above).

A number of explanations for the observed hierarchy in the ability of T cell epitopes to induce tolerance have been proposed (reviewed in Anderton and Wraith (1998) as above). In particular, it has been proposed that there is a correlation between the affinity of the peptide for the MHC and tolerogenicity (Liu and Wraith (1995) as above), but this does not tally with some of the observations. For example, MBP [89-101], which is not tolerogenic, binds to I-A^{S} with relatively high affinity. It is thus not straightforward to predict which peptides will induce tolerance.

The present inventors have shown that if a peptide epitope is of an appropriate size to be presented by immature APC without antigen processing, it can induce immunological tolerance (International patent application number PCT/GB01/03702). The observation that some T cell epitopes are tolerogenic and others are incapable of inducing tolerance can therefore be explained by the fact that some epitopes require antigen processing before they are capable of being presented by an MHC molecule. These epitopes which require further processing do not induce tolerance when administered in a soluble form, despite their capacity to induce disease when injected in combination with adjuvant.

The epitopes which do not require further processing are capable of inducing tolerance, and have been termed "apitopes" (Antigen Processing Independent epiTOPES) by the inventors.

### Antigen Processing Independent Presentation Systems (APIPS)

The peptides of the present invention are capable of binding to an MHC molecule *in vitro* and being presented to a T cell without antigen processing.

It is possible to test whether a peptide is capable of binding to an MHC molecule without antigen processing using a "processing free" system. Such a system should be capable of presenting antigen via MHC molecules to T cells, but incapable of processing antigen. Thus peptides may be tested for their capacity to bind to an MHC molecule *in vitro* and being presented to a T cell without antigen processing using an antigen processing independent presentation system (APIPS).

Examples of APIPS include:
a) fixed APC (with or without antibodies to CD28);
b) Lipid membranes containing Class I or II MHC molecules (with or without antibodies to CD28); and
c) purified natural or recombinant MHC in plate-bound form (with or without antibodies to CD28).

It is known to use fixed APC to investigate T cell responses, for example in studies to investigate the minimal epitope within a polypeptide, by measuring the response to truncated peptides (Fairchild et al (1996) Int. Immunol. 8:1035-1043). APC may be fixed using, for example formaldehyde (usually paraformaldehyde) or glutaraldehyde.

Lipid membranes (which may be planar membranes or liposomes) may be prepared using artificial lipids or may be plasma membrane/microsomal fractions from APC.

In use, the APIPS may be applied to the wells of a tissue culture plate. Peptide antigens are then added and binding of the peptide to the MHC portion of the APIPS is detected by addition of selected T cell lines or clones. Activation of the T cell line or clone may be measured by any of the methods known in the art, for example via ³H-thymidine incorporation or cytokine secretion.

If a peptide is capable of being presented to a T cell by an APIPS, then it is capable of binding to the MHC molecule without antigen processing, and is an apitope.

### Tolerance

The peptides of the present invention are capable of inducing tolerance to proteolipid protein.

As used herein, the term "tolerogenic" means capable of inducing tolerance.

Tolerance is the failure to respond to an antigen. Tolerance to self antigens is an essential feature of the immune system, when this is lost, autoimmune disease can result. The adaptive immune system must maintain the capacity to respond to an enormous variety of infectious agents while avoiding autoimmune attack of the self antigens contained within its own tissues. This is controlled to a large extent by the sensitivity of immature T lymphocytes to apoptotic cell death in the thymus (central tolerance). However, not all self antigens are detected in the thymus, so death of self-reactive thymocytes remains incomplete. There are thus also mechanisms by which tolerance may be acquired by mature self-reactive T lymphocytes in the peripheral tissues (peripheral tolerance). A review of the mechanisms of central and peripheral tolerance is given in Anderton et al (1999) (Immunological Reviews 169:123-137).

Tolerance may result from or be characterised by the induction of anergy in at least a portion of CD4+ T cells. In order to activate a T cell, a peptide must associate with a "professional" APC capable of delivering two signals to T cells. The first signal (signal 1) is delivered by the MHC-peptide complex on the cell surface of the APC and is received by the T cell via the TCR. The second signal (signal 2) is delivered by costimulatory molecules on the surface of the APC, such as CD80 and CD86, and received by CD28 on the surface of the T cell. It is thought that when a T cell receives signal 1 in the absence of signal 2, it is not activated and, in fact, becomes anergic. Anergic T cells are refractory to subsequent antigenic challenge, and may be capable of suppressing other immune responses. Anergic T cells are thought to be involved in mediating T cell tolerance.

Peptides which require processing before they can be presented in conjunction with MHC molecules do not induce tolerance because they have to be handled by mature antigen presenting cells. Mature antigen presenting cells (such as macrophages, B cells and dendritic cells) are capable of antigen processing, but also of delivering both signals 1 and 2 to a T cell, leading to T cell activation. Apitopes, on the other hand, will be able to bind class II MHC on immature APC. Thus they will be presented to T cells without costimulation, leading to T cell anergy and tolerance.

Of course, apitopes are also capable of binding to MHC molecules at the cell surface of mature APC. However, the immune system contains a greater abundance of immature than mature APC (it has been suggested that less than 10% of dendritic cells are activated, Summers et al. (2001) Am. J. Pathol. 159: 285-295). The default position to an apitope will therefore be anergy/tolerance, rather than activation.

It has been shown that, when tolerance is induced, the capacity of antigen-specific CD4+ T cells to proliferate is reduced. Also, the production of IL-2, IFN-γ and IL-4 production by these cells is down-regulated, but production of IL-10 is increased. Neutralisation of IL-10 in mice in a state of peptide-induced tolerance has been shown to restore completely susceptibility to disease. It has been proposed that a population of regulatory cells persist in the tolerant state which produce IL-10 and mediate immune regulation (Burkhart et al (1999) Int. Immunol. 11:1625-1634).

The induction of tolerance can therefore be monitored by various techniques including:
(a) reduced susceptibility to contract the disease for which the peptide is a target epitope *in vivo*;
(b) the induction of anergy in CD4+ T cells (which can be detected by subsequent challenge with antigen *in vitro*);
(c) changes in the CD4+ T cell population, including
   (i) reduction in proliferation;
   (ii) down-regulation in the production of, for example, IL-2, IFN-γ and IL-4; and
   (iii) increase in the production of IL-10.

### Target diseases

The peptide of the invention may be used in the treatment and/or prevention of a disease.

The disease may be a demyelinating diseases, such as adrenoleukodystrophy, vanishing white matter disease, or multiple sclerosis (MS).

The peptides of the present invention are particularly useful in the treatment and/or prevention of multiple sclerosis (MS). Multiple sclerosis (MS) is a chronic inflammatory disease characterised by multiple demyelinating lesions disseminated throughout the CNS white matter and occurring at various sites and times (McFarlin and McFarland, 1982 New England J. Medicine 307:1183-1188 and 1246-1251). MS is thought to be mediated by autoreactive T cells.

### Pharmaceutical composition

In a second aspect, the present invention relates to a pharmaceutical composition comprising one or more peptide(s) of the first aspect of the invention.

The present inventors predict that, despite "bystander suppression" it may be necessary to target a number of different T cell clones in order to induce tolerance effectively. Hence a plurality of peptides may be administered to an individual in order to prevent or treat a disease.

The pharmaceutical composition may, for example comprise between 1 and 20 apitopes, for example 1 to 15, 2 to 8 or 4 to 6 apitopes.

Where there are two or more apitopes, the pharmaceutical composition may be in the form of a kit, in which some or each of the apitopes are provided separately for simultaneous, separate or sequential administration.

Alternatively (or in addition) if the pharmaceutical composition (or any part thereof) is to be administered in multiple doses, each dose may be packaged separately.

The pharmaceutical composition may comprise a therapeutically or prophylactically effective amount of the or each apitope and optionally a pharmaceutically acceptable carrier, diluent or excipient.

Also, in the pharmaceutical compositions of the present invention, the or each apitope may be admixed with any suitable binder(s), lubricant(s), suspending agent(s), coating agent(s), or solubilising agent(s).

### Administration

The peptide may be administered in soluble form in the absence of adjuvant.

The peptide may be administered intranasally, by a mucosal, subcutaneous or intradermal route.

Studies have shown that peptide, when given in soluble form intraperitoneally (i.p.), intravenously (i.v.) or intranasally (i.n.) or orally can induce T cell tolerance (Anderton and Wraith (1998) as above; Liu and Wraith (1995) as above; Metzler and Wraith (1999) Immunology 97:257-263).

Studies in mice have demonstrated that the duration of peptide administration required to induce tolerance depends on the precursor frequency of T cells in the recipient (Burkhart et al (1999) as above). In many experimental studies, it has been shown that repeated doses of peptide are required to induce tolerance (Burkhart et al (1999) as above). The exact dose and number of doses of peptide will therefore depend on the individual; however, in a preferred embodiment a plurality of doses is administered.

If a plurality of peptides is administered simultaneously, they may be in the form of a "cocktail" which is suitable for administration in single or multiple doses. Alternatively it may be preferable to give multiple doses but vary the relative concentrations of the peptides between doses.

In a preferred embodiment a "dose escalation" protocol may be followed, where a plurality of doses is given to the patient in ascending concentrations. Such an approach has been used, for example, for phospholipase A2 peptides in immunotherapeutic applications against bee venom allergy (Müller et al (1998) J. Allergy Clin Immunol. 101:747-754 and Akdis et al (1998) J. Clin. Invest. 102:98-106).

### EXAMPLES

The following examples serve to illustrate the present invention, but should not be construed as a limitation thereof. The invention particularly relates to the specific embodiments described in these examples

### EXAMPLE 1 - Investigation of hydrophilic sections of the Proteolipid protein (PLP) sequence

### Materials and Methods

### Antigens

Since PLP is a largely hydrophobic protein, it was necessary to use the hydrophilic portions of the sequence. To this end, hydropathicity studies were carried out and eight peptides were synthesized from the hydrophilic domains of the PLP molecule, as follows:
36-61 (26mer): **HEAL**TGTEKLIETYFSKNYQDYEYLI-NH2
88-119 (32mer): **EGFY**TTGAVRQIFGDYKTTICGKGLSATVTGG-NH2
104-135 (32mer): **KTTI**CGKGLSATVTGGQKGRGSRGQHQAHSLE-NH2
119-150 (32mer): **GQKG**RGSRGQHQAHSLERVCHCLGKWLGHPDK-NH2
179-206 (28mer):**TWTT**CQSIAFPSKTSASIGSLCADARMY-NH2
192-219 (28mer): **TSAS**IGSLCADARMYGVLPWNAFPGKVC-NH2
207-234 (28mer): **GVLP**WNAFPGKVCGSNLLSICKTAEFQM-NH2
260-276 (17mer): **ATYN**FAVLKLMGRGTKF-NH2

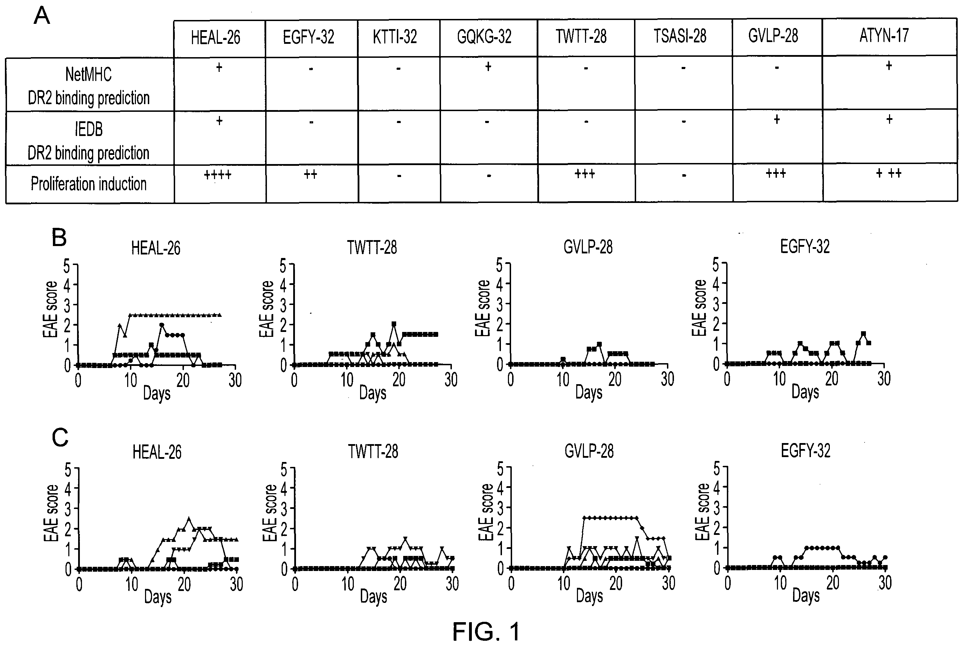

For each peptide, *in silico* studies were carried out to predict DR2 (HLA-DRB1*1501) binding capacity, and *in vitro* (proliferation assay) and *in vivo* (EAE induction) studies were carried out to investigate response. The results are shown in Figure 1.

Three peptides were shown to respond in both the *in vitro* and *in vivo* studies, and this correlated to the DR2 binding prediction. These peptides were HEAL-26, TWTT-28 and GVLP-28 (using the first four amino acids shown in bold in the sequences above to identify the peptide).

### Example 2 - Identification of Apitopes within HEAL-26

A panel of 15-mer overlapping peptides spanning HEAL-26 was synthesized using standard F-moc chemistry. Each peptide was displaced by 1 amino acid, as shown below:

| **HEAL-26 peptide** | **Sequence** |
|---|---|
| POP-1 | HEALTGTEKLIETYF |
| POP-2 | EALTGTEKLIETYFS |
| POP-3 | ALTGTEKLIETYFSK |
| POP-4 | LTGTEKLIETYFSKN |
| POP-5 | TGTEKLIETYFSKNY |
| POP-6 | GTEKLIETYFSKNYQ |
| POP-7 | TEKLIETYFSKNYQD |
| POP-8 | EKLIETYFSKNYQDY |
| POP-9 | KLIETYFSKNYQDYE |
| POP-10 | LIETYFSKNYQDYEY |
| POP-11 | IETYFSKNYQDYEYL |
| POP-12 | ETYFSKNYQDYEYLI |

The peptides were analysed using HEAL-26 specific hybridomas from DR2 mice. Of these peptides, POP-4, POP-7 and POP-8 were identified as apitopes.

### Example 3- Identification of Apitopes within TWTT-28

A panel of 15-mer overlapping peptides spanning TWTT-28 was synthesized using standard F-moc chemistry. Each peptide was displaced by 1 amino acid. Peptides were analysed using TWTT-28 specific hybridomas from DR2 mice.

The peptides POP-14 to POP-18 were identified as apitopes, having the following sequences:

| **TWTT-28 peptides** | **Sequence** |
|---|---|
| POP-14 | WTTCQSIAFPSKTSA |
| POP-15 | TTCQSIAFPSKTSAS |
| POP-16 | TCQSIAFPSKTSASI |
| POP-17 | CQSIAFPSKTSASIG |
| POP-18 | QSIAFPSKTSASIGS |

### Example 4- Identification of Apitopes within GVLP-28

A panel of 15-mer overlapping peptides spanning GVLP-28 was synthesized using standard F-moc chemistry. Each peptide was displaced by 1 amino acid. Peptides were analysed using TWTT-28 specific hybridomas from DR2 mice.

The peptide POP-22 was identified as an apitope, which has the following sequence: VLPWNAFPGKVCGSN.

### EXAMPLE 5 - Ex vivo tolerance assay

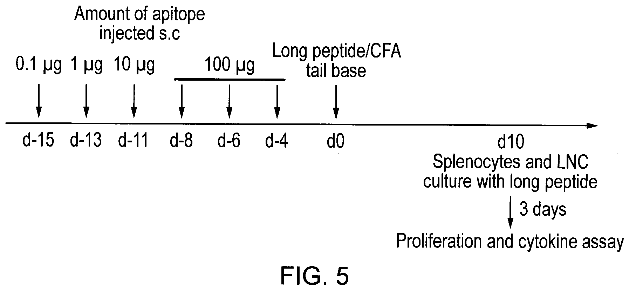

To assess the ability of the apitopes to induce tolerance, the inventors first determined the ability of these apitopes to inhibit an immune response *ex vivo.* For this purpose, HLA-DRB1*1501 mice were pre-treated with a dose escalation of an individual apitope and then primed with the corresponding long peptide. 10 days after priming, splenocytes (SPL) and lymph node cells (LNC) were cultured and stimulated with the corresponding long peptide for 3 days to assess their proliferation by ³H-thymidine incorporation, and the cytokine production by multiplex cytokine profiling systems (Figure 5).

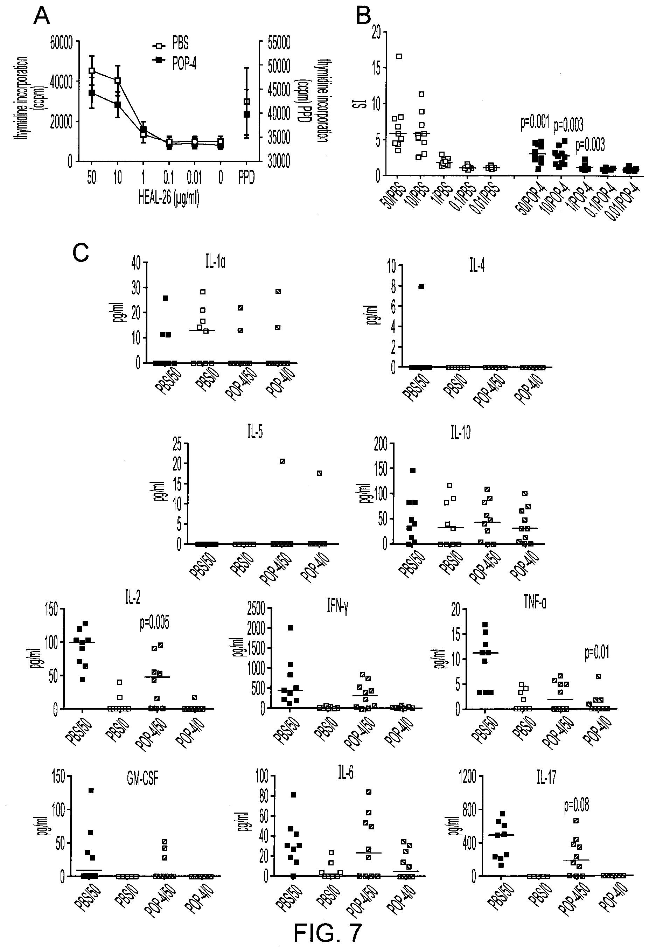

The tolerisation study shows thatPOP-4 pre-treatment induce an inhibition of T-cell proliferation and a suppression of Th1/Th17 cytokine production (significant decrease of IFN-γ, TNF-α and IL-17) in lymph node (Figure 6) and spleen (Figure 7). HEAL-26 (Figure 6 and 7) decrease the proliferation and decrease the production of IL-17 in both SPL and LNC. But an increase of IL-5 is also observed showing a shift from Th1/Th17 cytokine to Th2 cytokines when mice are pre-treated with HEAL-26.

POP-15 and TWTT-28 inhibit very well the proliferation and cytokine production of SPL and LNC. For POP-15, there is no difference in proliferation for the splenocytes but there are slight differences in cytokine production. Indeed, the mice treated with POP-15 produce less IFN-γ (statically significant), less GM-CSF and less IL-17. Moreover, there is a significant inhibition of proliferation and cytokines (IFN-γ, GM-CSF and IL-17) within the LNC when the mice are treated with POP-15.

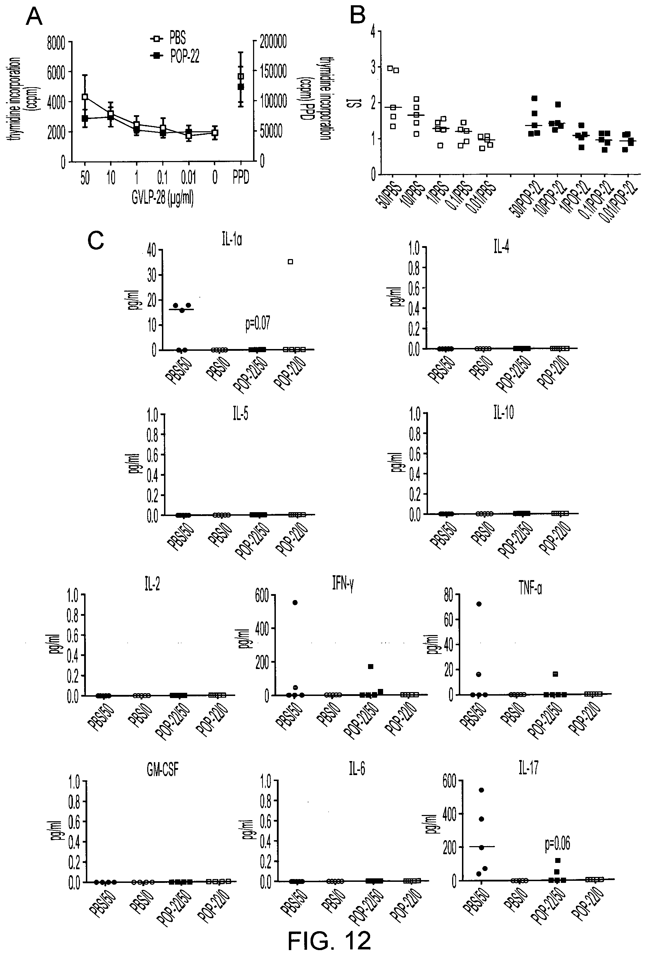

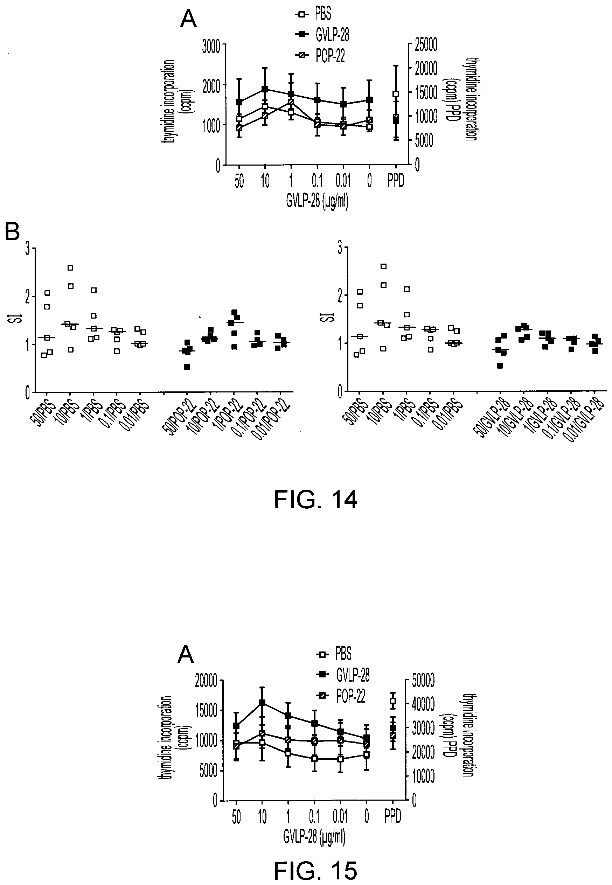

For POP-22, the results are less clear because of a very low response of PBS mice. However, it is possible to observe an effect of a pre-treatment with POP-22 and GVLP-28. Indeed, the stimulation index shows a decrease in LNC proliferation with POP-22 (Figure 12 and 14) and GVLP-28 (Figure 14). A significant inhibition of IL-17 production by these cells is also observed when the mice are pre-treated with POP-22 or GVLP-28.

### Material and Methods

### Mice

HLA-DRB1*1501 mice (DR2 mice) were obtained from Lars Fugger (LS Madsen et al. A humanized model for multiple sclerosis using HLA-DR2 and a human T-cell receptor. Nature genet 1999. 23, 343-347) and backcrossed into Ab⁰ mice. The resultant DR2 mice express the HLA-DRB1*1501 molecule but not the mouse MHC molecule.

### Peptides

Long peptides and 15-mer peptides were synthesised by GL Biochem Ltd (Shangai, China) and stored in dimethyl sulfoxide (DMSO, Sigma-Aldrich, Saint Louis, MA) at -80°C.

### investigation of peptides binding to HLA-DRB1*1501

### NetMHCII 2.2 Server

NetMHCII 2.2 server predicts binding of peptides to HLA-DRB1*1501 using artificial neuron networks. The prediction values are given in nM IC50 values. Strong and weak binding peptides are indicated in the output. High affinity binding peptides have an IC50 value below 50 nM, and weak binding peptides an IC50 values below 500 nM. The result is presented as prediction score which is calculated as follows: 1-log50000(aff). Website address: http://www.cbs.dtu.dk/services/NetMHCII.

### Immune Epitope DataBase (IEDB): consensus method

For each peptide, a percentile rank for each of the four methods (ARB, combinatorial library, SMM_align and Sturniolo) was generated by comparing the peptide's score against the scores of five million random 15 mers selected from SWISSPROT database. A small numbered percentile rank indicates high affinity. The median percentile rank of the four methods was then used to generate the rank for consensus method. Website address: http://tools.immuneepitope.org/analyze/html/mhc_II_binding.html.

### Determination of immunogenicity of the long peptides.

### Priming and EAE (Experimental autoimmune encephalomyelitis)

HLA-DRB1*1501 transgenic mice were injected with 100 µl containing 100 µg of long peptide in PBS (Lonza, Verviers, Belgium) or PBS alone, and with Complete Freund Adjuvant (CFA; BD Difco, Oxford, UK) with 4 mg/ml Mycobacterium tuberculosis (MTb, BD Difco, Oxford, UK) subcutaneously at the base of the tail. For EAE study, the mice were injected with 200ng of pertussis toxin at the same time as the priming and 2 days after. The mice were then followed, weight and scored for disease every day.

### Cell culture

On day 10, the draining lymph node and spleen were removed and splenocytes and lymph node cells isolated and cultured in X-vivo 15 medium (supplemented with glutamine, penicillin and streptomycin; Lonza, Verviers, Belgium) in 96-well flat bottom plates. 0.5x10⁶ cells/well in 200 µl/well were cultured with different concentrations of peptide for the proliferation assay.

### Proliferation assay and cytokine analysis

After 3 days in culture, 60 µl of supernatant was harvested (without disturbing the cells) and frozen. 25 µl/well of tritiated thymidine of the 20 µCi/ml pre-dilution (stock 5 mCi; PerkinElmer,Waltham, MA) were added to the cells to a final concentration of 0.5 µCi/well. The cells were incubated at 37°C. After 18 h, plates were frozen. Then thawed plates were harvested and read with β-counter (Wallac 1450 MicroBeta TriLux Liquid Scintillation Counter). The supernatant was then analysed with the mouse Th1/Th2 10plex FlowCytomix Multiplex (Bender).

### Generation of T cell clones

### Priming and T cell line establishment:

On day 0, five HLA-DRB1*1501 transgenic mice were injected with 100 µg of the long peptide in CFA with 4 mg/ml Mycobacterium tuberculosis at the base of the tail. A PBS primed control group was used as control for priming. On day 10, the draining lymph nodes and spleens were removed and splenocytes and lymph node cells isolated. Splenocytes and lymph node cells were mixed and the CD4 T cells purified using negative purification kit (untouched CD4 T cells; Miltneyi, Bergisch Gladbach, Germany). CD4 T cells were then restimulated with irradiated splenocytes (3000 rad) as APC (Antigen presenting cells) from HLA-DRB1*1501 mice at a ratio 1:1 at approximately 5x10⁶cells/ml and with a long peptide at 10 µg/ml in a 6-well plate. The stimulation was done in X-vivo 15 medium to avoid activating cells specific for foetal calf serum (FCS). On day 4, 20 U/ml of recombinant human IL-2 (R&D, Mineapolis, MN) was added to the cells. On day 7, all cells were harvested and dead cells were eliminated by Ficoll density gradient separation (Histopaque 1083, Sigma-Aldrich, St Louis, MA). Cells were then restimulated with irradiated splenocytes from DR2 mice with a ratio of APC:CD4 T cells at 2:1. Again the long peptide was added to the culture at 10 µg/ml. This time RPMI-5% FCS (Biosera, Ringmer, UK; supplemented with hepes, penicillin, streptomycin, glutamin: Lonza; and β-mercaptoethanol: gibco) was used. On day 7, cells were harvested, ficolled and fused.

### Fusion:

1x10⁷ BW5147 cells (Health Protection Agency Culture Collections, Salisbury, UK) and 1x10⁶ CD4 T cell line were mixed together and washed in 37°C serum free medium in a 50 ml tube using highest break setting on centrifuge for a firm pellet. The supernatant was poured off and the excess removed with a pipette. Cells were left in a water bath for 5 min to let the remaining medium drip down and then removed with a pipette. The cell pellet was gently but thoroughly resuspended. Then 1 ml of 37°C PEG (polyethylene glycol, 40-50% solution, Sigma-Aldrich, St Louis, MA) was added over 45 sec, keeping the cells in a mini water bath in the hood. The cells were incubated at 37°C for 45 sec. 1 ml of 37°C serum free medium was added over 30 sec while swirling tube, then 2 ml were added in the same way and then 3, 4, 10 and 30 ml were also added as above. The tube was inverted very slowly and incubated at 37°C for 5 min. Cells were then centrifuged for 5 min at 1300 rpm at room temperature (RT), without brake. The supernatant was carefully removed with a pipette (approximately 1 ml was left above the pellet). 50 ml of RT serum free medium was added without dislodging the cell pellet. The cells were centrifuged down as above and the wash was repeated with complete medium. Then cells were resuspended in 50 ml RT complete medium 10%-FCS and plated out in four 96-well flat bottom plates (100 µl /well). 38 ml of complete RPMI-10% FCS were then added to the tube and the previous step repeated twice to end with 3 series of dilution (12 plates in total) incubated at 37°C. After 48h, 100 µl of 2x HAT (Hypoxanthine-aminopterin-thymidine, Sigma-Aldrich, Saint Louis, MA) medium were added to each well. By day 6 hybridomas started to appear. Clones were maintained in HAT 1X medium until they were stable, then weaned into HT (Hypoxanthine-thymidine Sigma-Aldrich, Saint Louis, MA) medium for 2-3 weeks and then into complete RPMI. Clones were regularly frozen as they may become unstable (90% FCS+10%DMSO).

### Assessment of antigen-specificity of clones

100 µl of hybridoma cells were cultured with 5x10⁴ MGAR cells (human cell line expressing HLA-DRB1*1501, Health Protection Agency Culture Collections, Salisbury, UK) in wells of a flat bottom 96-well plate. 50 µl complete RPMI-10%FCS containing a long peptide at 10 µg/ml or the same volume of DMSO (diluent of the peptide) was added to the wells. After 48h, 120 µl of supernatant was removed and a mouse IL-2 ELISA (Enzyme-linked immunosorbent assay) performed. Clones that produce IL-2 recognise the antigen used. The remaining supernatants were frozen at -20°C.

### IL-2 ELISA

96 well plates (Immunosorb 96 well, Nunc, Roskilde , Denmark) were coated with 50µl/well purified rat anti-mouse IL-2 capture Ab (BD Biosciences, Oxford, UK), diluted 1:250 in carbonate buffer (3.56g Na2CO3 (Sigma-Aldrich, Saint Louis, MA), 8.4g NaHCO3 (Fisher Scientific, Loughborough, UK), 1L elgastat water; pH 9.5) and incubated overnight at 4°C. After 2 washes in PBS-Tween (1L 10x PBS, 9L distilled water, 0.5 ml Tween (Sigma-Aldrich, Saint Louis, MA)), 200µl/well of PBS-10% FCS were added and incubated at RT for 1 hour. After 3 washes in PBS-Tween, 50 µl of supernatant or IL-2 standard (BD Biosciences, Oxford, UK) dilutions (in PBS-10% FCS) were added to the wells and incubated 2h at RT. After 4 washes in PBS-Tween (Sigma-Aldrich, Saint Louis, MA), 50µl/well of biotin rat anti-mouse IL2 (BD Biosciences, Oxford, UK) diluted 1:1000 in 10% FCS/PBS was added and incubated for 1 h at RT. After 4 washes, 50µl/well extravidin peroxidase (Sigma-Aldrich, Saint Louis, MA) diluted 1:1000 in PBS was added and incubated 30 min at RT. After 4 washes, 50µl/well of substrate solution* was added and incubated at RT until a clear colour change was seen. The reaction was stopped using 50µl/well 2M H₂SO₄ (BDH, Poole, UK) and plates were read at 450 nm (550nm ref) with an ELISA reader (SpectraMax Pro, Molecular Device, Sunnyvale, CA). *Substrate solution: 10 ml phosphate-citrate buffer 0.1M (5.14 ml 0.2M Na2HPO4 (BDH, Poole, UK), 4.86 ml 0.1M citrate (Sigma), 10 ml elgastat water), 0.1 ml of TMB (defrost 3,3',5,5'-Tetramethylbenzidine at 10mg/ml in DMSO, Sigma-Aldrich, Saint Louis, MA), 6 µl of hydrogen peroxide (Sigma-Aldrich, Saint Louis, MA).

### Antigen processing independent presentation system

The specific clones were then tested for their responses to the 15-mer peptides (POP-1 to POP-12) with fixed or not fixed MGAR cells. For this purpose, 1x10⁵ cells from the individual clones are cultured with 5x10⁴ fixed or fresh MGAR cells in a 96-well flat bottom plate. For the MGAR cell fixation, 20x10⁶ MGAR cells were incubated for 5 min with 6 ml of Paraformaldehyde (PFA, BDH, Poole, UK) 0.5% (pH7) at RT and then 6 ml of Glycine (Fisher Scientific, Loughborough, UK) at 0.4M was added to stop the reaction. The cells were then washed and resuspended in RPMI-10%FCS. 10µg/ml of each 15-mer peptide diluted in RPMI-10%FCS was added to individual wells. A well containing DMSO instead of peptide was used for each clone as a negative control and a well containing the long peptide was used as a positive control. After 48h in culture, 120 µl of supernatant was harvested and analysed by ELISA to assess IL-2 production.

### Tolerance induction with apitope treatment

HLA-DRB1*1501 transgenic mice were pre-treated with a dose escalation (0.1, 1, 10 and 3 times 100 µg) of apitope or 100 µl of PBS at day -15, -13, -11, -8, -6, -4. On day 0, the mice were primed with 100 µg of the long peptide in CFA with 4 mg/ml Mycobacterium tuberculosis at the base of the tail. After 10 days, the inguinal lymph nodes and the spleen were harvested. The proliferation and cytokine production by LNC and splenocytes are then analysed as described above.

Various modifications and variations of the described methods and system of the invention will be apparent to those skilled in the art.

Although the invention has been described in connection with specific preferred embodiments, it should be understood that the invention as claimed should not be unduly limited to such specific embodiments, Indeed, various modifications of the described modes for carrying out the invention which are obvious to those skilled in chemistry or molecular biology or related fields are intended to be covered by the present invention.

## Claims

1. A peptide which is capable of binding to an MHC molecule *in vitro* and being presented to a T cell without antigen processing, and which is selected from the following proteolipid protein (PLP) peptides:
PLP 39-53: LTGTEKLIETYFSKN (SEQ ID NO. 7)
PLP 42-56: TEKLIETYFSKNYQD (SEQ ID NO. 8) and
PLP 43-57: EKLIETYFSKNYQDY (SEQ ID NO. 9).

2. A peptide according to claim 1, for use in the treatment and/or prevention of a demyelinating disease.

3. The peptide for use according to claim 2, wherein the disease is multiple sclerosis.

4. A pharmaceutical composition comprising one or more peptides according to claim 1.

## Patentansprüche

1. Peptid, welches dazu in der Lage ist, in vitro an ein MHC-Molekül zu binden und einer T-Zelle ohne Antigen-Prozessierung präsentiert werden kann und welches aus den folgenden Proteolipidprotein(PLP)-Peptiden ausgewählt ist:
PLP 39-53: LTGTEKLIETYFSKN (SEQ ID NO: 7)
PLP 42-56: TEKLIETYFSKNYQD (SEQ ID NO: 8) und
PLP 43-57: EKLIETYFSKNYQDY (SEQ ID NO: 9).

2. Peptid nach Anspruch 1 zur Verwendung bei der Behandlung und/oder Prävention einer demyelinisierenden Krankheit.

3. Peptid zur Verwendung nach Anspruch 2, wobei es sich bei der Krankheit um multiple Sklerose handelt.

4. Pharmazeutische Zusammensetzung, umfassend ein oder mehrere Peptide nach Anspruch 1.

## Revendications

1. Peptide qui est capable de se lier à une molécule du CMH *in vitro* et d'être présenté à un lymphocyte T sans traitement de l'antigène, et qui est choisi parmi les peptides de protéines protéo-lipidiques (PLP) suivants :
PLP 39-53 : LTGTEKLIETYFSKN (SEQ ID n°: 7)
PLP 42-56 : TEKLIETYFSKNYQD (SEQ ID n° : 8) et
PLP 43-57 : EKLIETYFSKNYQDY (SEQ ID n° : 9).

2. Peptide, selon la revendication 1, destiné à être utilisé dans le traitement et/ou la prévention d'une maladie démyélinisante.

3. Peptide destiné à être utilisé selon la revendication 2, dans lequel la maladie est la sclérose en plaques.

4. Composition pharmaceutique comprenant un ou plusieurs peptide(s) selon la revendication 1.
